# EUROPEAN PATENT SPECIFICATION

(11) **EP 4 100 162 B1**
(45) Date of publication and mention of the grant of the patent: **30.10.2024**
(21) Application number: 21745253.1
(22) Date of filing: 02.07.2021
(51) Int. Cl.: B01L 3/00, G01N 33/49

(54) **FUNCTIONALIZED BLOOD SAMPLING DEVICE AND METHOD FOR PETH MEASUREMENT**
FUNKTIONALISIERTES BLUTPROBENAHMEGERÄT UND METHODE FÜR DIE PETH-MESSUNG
DISPOSITIF FONCTIONNALISE DE PRELEVEMENT DE SANG ET PROCEDE DE MESURE DE PETH

(30) Priority: 02.07.2020 SE 2050826
(43) Date of publication of application: 14.12.2022
(73) Proprietor: Capitainer AB, 171 45 Solna (SE)
(72) Inventor: BECK, Olof, 132 44 Saltsjö-Boo (SE)
(74) Representative: Bergenstråhle & Partners AB
(86) International application number: PCT/EP2021/068383
(87) International publication number: WO 2022/003177

(56) References cited:
- US-A1- 2010 004 170
- US-A1- 2012 041 481
- UGWUANYI DANIEL C ET AL: "Biochemical, histopathological and morphological alterations in the kidneys of wistar rats following exposure to X-ray film developer solutions", EUROPEAN JOURNAL OF EXPERIMENTAL BIOLOGY, vol. 6, 1 January 2016 (2016-01-01), pages 43 - 52, XP093061215
- CAMPBELL D. M. ET AL: "Colorimetric determination of glucose in 20 l. of blood", JOURNAL OF CLINICAL PATHOLOGY, vol. 16, no. 2, 1 March 1963 (1963-03-01), GB, pages 173 - 174, XP093093737, ISSN: 0021-9746, Retrieved from the Internet <URL:https://jcp.bmj.com/content/jclinpath/16/2/173.full.pdf> DOI: 10.1136/jcp.16.2.173
- BECK OLOF ET AL: "Study of measurement of the alcohol biomarker phosphatidylethanol (PEth) in dried blood spot (DBS) samples and application of a volumetric DBS device", CLINICA CHIMICA ACTA, vol. 479, 1 April 2018 (2018-04-01), AMSTERDAM, NL, pages 38 - 42, XP055852542, ISSN: 0009-8981, DOI: 10.1016/j.cca.2018.01.008
- SCHRÖCK ALEXANDRA ET AL: "Determination of the formation rate of phosphatidylethanol by phospholipase D (PLD) in blood and test of two selective PLD inhibitors", ALCOHOL, vol. 73, 1 December 2018 (2018-12-01), AMSTERDAM, NL, pages 1 - 7, XP055852638, ISSN: 0741-8329, DOI: 10.1016/j.alcohol.2018.03.003
- BECK OLOF ET AL: "Measurement of the alcohol biomarker phosphatidylethanol (PEth) in dried blood spots and venous blood-importance of inhibition of post-sampling formation from ethanol", ANALYTICAL AND BIOANALYTICAL CHEMISTRY, SPRINGER BERLIN HEIDELBERG, DE, vol. 413, no. 22, 15 February 2021 (2021-02-15), pages 5601 - 5606, XP037554243, ISSN: 1618-2642, [retrieved on 20210215], DOI: 10.1007/S00216-021-03211-Z

## Description

### Technical field

The present invention relates to improvements in sampling of blood to determine the alcohol biomarker phosphatidylethanol (PEth). More specifically, the invention relates to a device and method for collection and subsequent testing for PEth of a blood sample.

### Background art

Phosphatidylethanol is a substance that forms from cell membrane phospholipids during exposure to ethanol (i.e. alcohol drinking). Phospholipase D (PLD) is the enzyme responsible for the reaction and is caused by the feature of the enzyme to prefer ethanol as substrate over water when acting to form phosphatidic (PA) acid from phosphatidylcholines (PCs) as part of a regulatory system. When formed, PEth will be incorporated into the cell membrane and becomes accumulated over time. Repeated alcohol drinking will lead to elevated blood levels of PEth and it has been suggested as biomarker to detect riskful alcohol consumption from risk concluding limits. For this purpose, several assays have been suggested in for example US 5,066,583; WO 2009/054784; US 8,795,980 and US 2019/0293668.

Sampled blood is an active biofluid that retains many biological activities after sampling. One such activity is the enzyme Phospholipase D (PLD), which is involved in regulation of the lipid content of the cell membrane. PLD has the unique feature to have activity also after cooling and freezing. Accordingly, PEth can be formed *in vitro* post sampling if ethanol is present in the blood at the time of sampling, which is not uncommon in real practice.

O Beck et al in Clinica Chimica Acta, 2018, Vol. 479, pages 28-42 discloses using dried blood spot (DBS) samples collected on filter paper by means of a volumetric DBS device for PEth measurement.

A Schröck et al in Alcohol, 2018, Vol. 73, pages 1-7 discloses the development of standardized tests for measuring individual formation of PEth following alcohol consumption. The authors were aware of the problem with post-sampling formation of PEth and introduced the well-known PLD inhibitors halopemide and FIPI (5-fluoro-2-indolyl-deschlorohalopemide) to improve the quality of the PEth analysis in order to prevent *in vitro* formation of PEth in alcohol positive blood samples after blood sampling. However, halopemide and FIPI were considered unsuitable for clinical use due to low potency and high costs. For this reason, the problem with post-sampling PLD needs to be solved before feasible routine assays of PEth can be established.

UGWUANYI DANIEL C ET AL: "Biochemical, histopathological and morphological alterations in the kidneys of wistar rats following exposure to X-ray film developer solutions", EUROPEAN JOURNAL OF EXPERIMENTAL BIOLOGY, vol. 6, 1 January 2016 (2016-01-01), pages 43-52 discloses assessing the alterations in serum urea and serum creatinine levels from a centrifuged mixture of serum, distilled water, NH2SO4 and a 10% solution of sodium tungstate.

CAMPBELL D. M. ET AL: "Colorimetric determination of glucose in 20 I. of blood",JOURNAL OF CLINICAL PATHOLOGY, vol. 16, no. 2, 1 March 1963 (1963-03-01), pages 173-174 describes a method for reducing ultra micro copper by using a method wherein sodium tungstate is used with copper sulphate to precipitate proteins from blood samples.

In addition, it is desirable to conform routine PEth assay to the economic and rapid screening technology admitted by DBS (Dried Blood Spot Sampling) where blood samples are conveniently collected and dried, as represented by the device disclosed by US2015/0037903. Although, it was estimated that dried blood spots may solve the problems with post-sampling formation of PEth, it was found that PLD remains active and that PEth forms also during the drying process and possibly also in the dried blood. There is an obvious need to find a solution to the possibility of post-sampling formation of PEth as it may lead to erroneous measurements and false interpretations.

The present invention therefore meets need of finding better strategies to develop a reliable, economic and convenient assay of PEth.

### Summary of invention

An object of the present invention is thus to provide a simple solution to a reliable, economical and convenient assay of PEth that avoids the problems with post-sampling formation of PEth. The invention is set out in the appended claims.

In one aspect of the invention, there is provided a device configured for collection and subsequent testing for phosphatidylethanol (PEth) of a blood sample of less than 10 ml. The device is a test tube and includes at least one inhibitor of the enzyme phospholipase D (PLD) selected from at least one of a salt comprising a vanadium oxyanion and a salt comprising a tungsten oxyanion. The PLD inhibitor is immobilized in the test tube.

The inhibitor of the enzyme phospholipase D may be selected from at least one of NaVOs (sodium metavanadate) and Na₂WO₄ (sodium tungstate).

The at least one salt of inhibitor of the enzyme phospholipase D may have a mass of less than 10 mg.

The at least one salt is present in amount that admits concentration of less than 1 mM in the blood sample.

The PLD inhibitor may be present in a gel attached to the walls of the test tube.

The walls of the test tube may be impregnated with the PLD inhibitor.

The walls of the test tube may have a coating, e.g. of less than 100 micrometers, comprising the at least one inhibitor of the enzyme phospholipase D.

The inner coating may be sprayed on the on the bottom and/or on the lid of the test tube.

In another aspect of the invention, a method is disclosed for preparing a sample for analysis of phosphatidylethanol (PEth) the method comprising adding a blood sample from a patient with a volume of less than 10 ml to a test tube (102), wherein the test tube includes at least one inhibitor of the enzyme phospholipase D (PLD) selected from at least one of a salt comprising a vanadium oxyanion and a salt comprising a tungsten oxyanion; contacting the blood sample with the PLD inhibotor; and admitting inhibition of the PLD in the blood sample with the at least one PLD inhibitor so formation of PEth is blocked.

### Brief description of drawings

The invention is now described, by way of example, with reference to the accompanying drawing, in which:

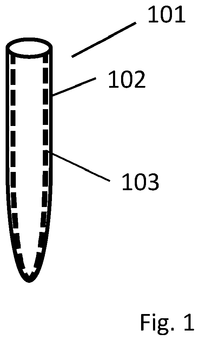

Fig.1 shows a test tube device according to the invention.

### Description of embodiments

In the following, a detailed description of embodiments of the invention is disclosed.

Fig. 1 shows a device 101 according to the present invention, comprising a blood collection test tube 102 that contains an PLD inhibitor enzyme. The blood collection test tube can be a conventional blood test tube made of glass or suitable polymers, such as Vacutainer^{®}, specially labelled for PEth analysis. Similar to the blood collection test tubes with added components such as EDTA, heparin, etc. that are already known in the art, the test tube comprises an immobilized PLD inhibitor, as an added component.

In one alternative, the PLD inhibitor enzyme may be present in a gel attached to the walls of the test tube. Other means of immobilizing the PLD inhibitor enzyme are also contemplated.

It has been found that the salts of a transition metal belonging to column 5 or 6 of the periodic table, in particular, the salts of vanadium and tungsten are effective inhibitors of the enzyme phospholipase D. The PLD inhibitor is selected from at least one of a salt comprising a vanadium oxyanion and a salt comprising a tungsten oxyanion, preferably NaVOs (sodium metavanadate) or Na₂WO₄ (sodium tungstate).

Other agents that may also be used as inhibitors of the enzyme D may include certain anti-cancer drugs, such as 5-fluoro-2-indolyldes-chlorohalopemide (FIPI), or agents with certain ionic properties, such as phosphate analogues.

In further embodiments, the walls of the test tube may have been impregnated with a PLD inhibitor, or the walls of the test tube may have a coating comprising the PLD inhibitor. The PLD inhibiting agents can be included in thin film coatings of the container (see for example US 6428527) or added by other conventional ways to supply blood test tubes with additives.

An exemplary method of applying a coating of at least one inhibitor of the enzyme phospholipase D to a test tube is disclosed. The method comprises the steps of stabilizing the test in a substantially vertical position; inserting a spray nozzle inside the test tube, the spray nozzle being in fluid connection to a container holding a solution comprising at least one inhibitor of the enzyme phospholipase D; spraying the solution comprising at least one inhibitor of the enzyme phospholipase D inside the test tube; and allowing the solution comprising at least one inhibitor of the enzyme phospholipase D to dry.

In addition to the PLD inhibitor, the test tube may further include other conventional additives such as anticoagulants.

The test tube is used for blood collection. Approximately 5 -10 ml of blood is added to the test tube. The test tube is then sealed/closed with a test tube cap and the now sealed test tub is gently rocked for approximately 5 minutes. Thereafter, the sealed test tube containing the blood sample is stored at either room temperature or +4°C pending further analysis.

### Example 1

### Blood samples

The blood specimens used for the present investigation were deidentified surplus volumes of venous whole blood selected among those sent to the Department of Clinical Pharmacology, Karolinska University Laboratory (Stockholm) for routine analysis of PEth as an alcohol biomarker.

Additional blank specimens were collected from non-drinking healthy volunteers at the laboratory. The blood was collected in EDTA tubes and stored at 4 °C where PEth is reported to be stable for at least 3 weeks. Ethanol was not tested for. The procedures followed were approved by the ethics committee at the Karolinska University Hospital (No. 2013/341-31/4).

### Measurement of PEth in fresh venous blood

Routine analysis of PEth 16:0/18:1 in whole blood specimens was done essentially as previously described. 100 µL whole blood was mixed with 50 µL IS solution (PEth-d31; Avanti Polar Lipids, Alabaster, AL, USA), 75 µL acetonitrile, and 150 µL acetone. The mixture was gently shaken (40 rpm) for 20 min at room temperature and then centrifuged at 4000g for 20 min. The supernatant was transferred to a new vial and centrifuged again for another 10 min. LC-MS/MS quantification of PEth 16:0/18:1 was done by comparison with a calibration curve covering 0-14.2 µmol/L prepared similarly in blank (i.e. PEth negative) blood specimens spiked with known amounts of PEth 16:0/18:1 (Avanti Polar Lipids) [14]. Two quality control samples (low and high PEth level) were prepared in the same way. The detection limit (LOD) and lower quantification limit (LLOQ) of the method were 0.01 µmol/L and 0.03 µmol/L, respectively.

### Measurement of PEth in DBS collected on standard filter paper

For method evaluation and validation, DBS samples were prepared by pipetting 45 µL of the venous blood specimen onto the filter paper (Whatman 903 Protein Saver Card; GE Healthcare Ltd., Cardiff, UK), which was then allowed to dry at room temperature for at least 3 hours in horizontal position with air on both sides. For the analysis, 3 filter paper punches (4.7 mm diameter) were taken from each DBS and placed in a test tube and added with 10 µL IS solution and 125 µL methanol. The tube was covered and gently shaken (40 rpm) for 1 hour, followed by centrifugation at 4000 g for 10 min. The liquid phase was transferred to an autosampler vial and centrifuged for another 10 min, prior to analysis. LC-MS/MS quantification of PEth 16:0/18/1 in the sample was done by comparison with a DBS calibration curve prepared as described for venous blood.

### Measurement of PEth in DBS samples collected using a volumetric device

The value of volumetric DBS measurement of PEth was examined using a disposable prototype DBS device (provided by Capitainer AB, Stockholm, Sweden) [25] constructed with an inlet cavity for applying 30-50 µL (i.e. about one drop) of blood. After the application of blood, a capillary channel is automatically filled with 14 µL of the sample which is eventually emptied onto a Whatman 903 filter paper disc (6.0 mm diameter). The paper disc is impregnated with, for example, 1 mg of salt. For this study, 50 µL venous whole blood was applied to the inlet cavity using a pipette.

After drying at room temperature for at least 2 hours, the volumetric filter disc was transferred to a glass test tube and extracted with 200 µL isopropanol containing IS (0.035 µmol/L PEth-d5; Chiron AS, Trondheim, Norway). The test tube was gently shaken (40 rpm) for 60 min at room temperature and 150 µL of the liquid phase transferred to an autosampler vial. A 2-µL aliquot was injected on the column and the PEth 16:0/18:1 concentration was measured by LC-MS/MS as described above. The standards were prepared by fortifying DBS spots from blank blood with reference PEth 16:0/18:1 in isopropanol. The method was calibrated between 0.025 and 5.00 µmol/L PEth 16:0/18:1. Uncertainty in quantification was documented both for intra- and interassay imprecision and accuracy, using authentic blood quality controls with assigned PEth concentrations.

### Results:

**Table I**

| Experiment with PEth formation during drying on DBS filter paper | | |
|---|---|---|
| Blank blood + 2 per mille ethanol | PEth response native | PEth response with salt |
| Sample 1 | 33 | Not detected |
| Sample 1 | 29 | Not detected |
| Sample 2 | 33 | Not detected |
| Sample 2 | 39 | Not detected |
| Sample 3 | 30 | Not detected |
| Sample 3 | 24 | Not detected |

The results demonstrate that with the inhibitor present during drying additional artificial formation of PEth does not take place.

Thus, the use of the rock salts NaVOs and NA₂WO₄ is simple, easy to work with and safe. They withstand the temperature during production.

## Claims

1. A device (101) configured for collection and subsequent testing for phosphatidylethanol (PEth) of a blood sample of less than 10 ml, wherein the device is a test tube (102) and includes at least one inhibitor of the enzyme phospholipase D (PLD) selected from at least one of a salt comprising a vanadium oxyanion and a salt comprising a tungsten oxyanion, **characterized in that** the PLD inhibitor is immobilized in the test tube.

2. The device according to claim 1, wherein the PLD inhibitor is selected from at least one of NaVOs (sodium metavanadate) and Na₂WO₄ (sodium tungstate).

3. The device according to claim 1 or 2, wherein the at least one salt has a mass of less than 10 mg.

4. The device according to any one of the preceding claims, wherein the at least one salt is present in amount that admits concentration of less than 1 mM in the blood sample.

5. The device according to any one of the preceding claims, wherein the at least one PLD inhibitor is present in a gel attached to the walls of the test tube.

6. The device according to any one of claims 1-4, wherein the walls of the test tube are impregnated with the at least one PLD inhibitor.

7. The device according to any one of claims 1-4, wherein the walls of the test tube have a coating comprising the at least one PLD inhibitor.

8. The device according to any one of claims 1-4, comprising a bottom, a lid and an inner coating of less than 100 micrometers comprising the at least one PLD inhibitor.

9. The device according to claim 8, wherein the inner coating is sprayed on the bottom and/or on the lid of the test tube.

10. A method of preparing a sample for analysis of phosphatidylethanol (PEth) comprising:
adding a blood sample from a patient with a volume of less than 10 ml to a test tube (102), wherein the test tube includes at least one inhibitor of the enzyme phospholipase D (PLD) selected from at least one of a salt comprising a vanadium oxyanion and a salt comprising a tungsten oxyanion, wherein the PLD inhibitor is immobilized in the test tube;
contacting the blood sample with the PLD inhibitor; and
admitting inhibition of the PLD in the blood sample with the at least one PLD inhibitor so formation of PEth is blocked.

## Patentansprüche

1. Vorrichtung (101), die zur Entnahme und anschließenden Untersuchung auf Phosphatidylethanol (PEth) einer Blutprobe von weniger als 10 ml konfiguriert ist, wobei die Vorrichtung ein Reagenzglas (102) ist und mindestens einen Inhibitor des Enzyms Phospholipase D (PLD) beinhaltet, ausgewählt aus mindestens einem von einem Salz, das ein Vanadiumoxyanion umfasst, und einem Salz, das ein Wolframoxyanion umfasst, **dadurch gekennzeichnet, dass** der PLD-Inhibitor im Reagenzglas immobilisiert ist.

2. Vorrichtung nach Anspruch 1, wobei der PLD-Inhibitor aus mindestens einem von NaVO₃ (Natriummetavanadat) und Na₂WO₄ (Natriumwolframat) ausgewählt ist.

3. Vorrichtung nach Anspruch 1 oder 2, wobei das mindestens eine Salz eine Masse von weniger als 10 mg aufweist.

4. Vorrichtung nach einem der vorstehenden Ansprüche, wobei das mindestens eine Salz in einer Menge vorhanden ist, die eine Konzentration von weniger als 1 mM in der Blutprobe zulässt.

5. Vorrichtung nach einem der vorstehenden Ansprüche, wobei der mindestens eine PLD-Inhibitor in einem an den Wänden des Reagenzglases haftenden Gel vorliegt.

6. Vorrichtung nach einem der Ansprüche 1-4, wobei die Wände des Reagenzglases mit dem mindestens einen PLD-Inhibitor imprägniert sind.

7. Vorrichtung nach einem der Ansprüche 1-4, wobei die Wände des Reagenzglases eine Beschichtung aufweisen, die mindestens einen PLD-Inhibitor umfasst.

8. Vorrichtung nach einem der Ansprüche 1-4, die einen Boden, einen Deckel und eine innere Beschichtung von weniger als 100 Mikrometern umfasst, die den mindestens einen PLD-Inhibitor umfasst.

9. Vorrichtung nach Anspruch 8, wobei die innere Beschichtung auf den Boden und/oder auf den Deckel des Reagenzglases gesprüht ist.

10. Verfahren zur Vorbereitung einer Probe zur Analyse von Phosphatidylethanol (PEth), umfassend:
Hinzufügen einer Blutprobe eines Patienten mit einem Volumen von weniger als 10 ml in einem Reagenzglas (102), wobei das Reagenzglas mindestens einen Inhibitor des Enzyms Phospholipase D (PLD) beinhaltet, ausgewählt aus mindestens einem Salz, das ein Vanadiumoxyanion umfasst, und einem Salz, das ein Wolframoxyanion umfasst, wobei der PLD-Inhibitor im Reagenzglas immobilisiert ist;
Inkontaktbringen der Blutprobe mit dem PLD-Inhibitor; und
Zulassen einer Hemmung der PLD in der Blutprobe mit dem mindestens einen PLD-Inhibitor, so dass die Bildung von PEth blockiert wird.

## Revendications

1. Dispositif (101) configuré pour la collecte et le test ultérieur de phosphatidyléthanol (PEth) d'un échantillon de sang de moins de 10 ml, dans lequel le dispositif est un tube à essai (102) et inclut au moins un inhibiteur de l'enzyme phospholipase D (PLD) sélectionné parmi au moins l'un d'un sel comprenant un oxyanion de vanadium et d'un sel comprenant un oxyanion de tungstène, **caractérisé en ce que** l'inhibiteur de PLD est immobilisé dans le tube à essai.

2. Dispositif selon la revendication 1, dans lequel l'inhibiteur de PLD est sélectionné parmi au moins l'un du NaVO₃ (métavanadate de sodium) et du Na₂WO₄ (tungstate de sodium).

3. Dispositif selon la revendication 1 ou 2, dans lequel le au moins un sel présente une masse inférieure à 10 mg.

4. Dispositif selon l'une quelconque des revendications précédentes, dans lequel le au moins un sel est présent en une quantité qui admet une concentration inférieure à 1 mM dans l'échantillon de sang.

5. Dispositif selon l'une quelconque des revendications précédentes, dans lequel le au moins un inhibiteur de PLD est présent dans un gel fixé aux parois du tube à essai.

6. Dispositif selon l'une quelconque des revendications 1-4, dans lequel les parois du tube à essai sont imprégnées du au moins un inhibiteur de PLD.

7. Dispositif selon l'une quelconque des revendications 1-4, dans lequel les parois du tube à essai présentent un revêtement comprenant le au moins un inhibiteur de PLD.

8. Dispositif selon l'une quelconque des revendications 1-4, comprenant un fond, un couvercle et un revêtement interne de moins de 100 micromètres comprenant le au moins un inhibiteur de PLD.

9. Dispositif selon la revendication 8, dans lequel le revêtement interne est pulvérisé sur le fond et/ou sur le couvercle du tube à essai.

10. Procédé de préparation d'un échantillon pour l'analyse du phosphatidyléthanol (PEth) comprenant :
l'ajout d'un échantillon de sang d'un patient avec un volume de moins de 10 ml à un tube à essai (102), dans lequel le tube à essai inclut au moins un inhibiteur de l'enzyme phospholipase D (PLD) sélectionné parmi au moins l'un d'un sel comprenant un oxyanion de vanadium et d'un sel comprenant un oxyanion de tungstène, dans lequel l'inhibiteur de PLD est immobilisé dans le tube à essai ;
la mise en contact de l'échantillon de sang avec l'inhibiteur de PLD ; et
l'admission de l'inhibition du PLD dans l'échantillon de sang avec le au moins un inhibiteur de PLD de sorte que la formation de PEth soit bloquée.
